# EUROPÄISCHE PATENTSCHRIFT

(11) **EP 2 306 949 B1**
(45) Veröffentlichungstag und Bekanntmachung des Hinweises auf die Patenterteilung: **20.06.2012**
(21) Anmeldenummer: 08773775.5
(22) Anmeldetag: 30.06.2008
(51) Int. Cl.: A61F 9/008, A61F 9/01, A61B 3/10

(54) **VORRICHTUNG ZUR OPHTHALMOLOGISCHEN, INSBESONDERE REFRAKTIVEN LASERCHIRURGIE**
DEVICE FOR OPHTHALMOLOGIC, PARTICULARLY REFRACTIVE, LASER SURGERY
DISPOSITIF DE CHIRURGIE LASER, NOTAMMENT RÉFRACTIVE, EN OPHTALMOLOGIE

(43) Veröffentlichungstag der Anmeldung: 13.04.2011
(73) Patentinhaber: WaveLight GmbH, 91058 Erlangen (DE)
(72) Erfinder: RIEDEL, Peter, 90489 Nürnberg (DE); DONITZKY, Christof, 90542 Eckental (DE)
(74) Vertreter: Katérle, Axel
(86) Internationale Anmeldenummer: PCT/EP2008/005334
(87) Internationale Veröffentlichungsnummer: WO 2010/000280

(56) Entgegenhaltungen:
- EP-A- 1 602 321
- WO-A-01/19303
- US-A1- 2005 024 586

## Beschreibung

Die Erfindung betrifft eine Vorrichtung zur ophthalmologischen, insbesondere refraktiven Laserchirurgie.

Die Chirurgie des humanen Auges kennt zahlreiche Behandlungsmethoden, bei denen Laserstrahlung auf das Auge gerichtet wird, um infolge der Wechselwirkung der eingestrahlten Laserstrahlung mit dem Auge einen indizierten Behandlungszweck zu erreichen. Bei der refraktiven Laserchirurgie ist der Behandlungszweck eine Änderung der Abbildungseigenschaften des optischen Systems "Auge" mittels der Laserstrahlung. Da für die Abbildungseigenschaften des humanen Auges vor allem die Hornhaut (Kornea) maßgeblich ist, umfasst die refraktive Laserchirurgie des Auges in vielen Fällen eine Bearbeitung der Kornea. Durch gezieltes Einbringen von Schnitten und/oder durch gezielten Materialabtrag wird dabei eine Formänderung der Kornea bewirkt; man spricht deshalb auch von einer Neuformung.

Ein bekanntes Beispiel einer Neuformung der Kornea zur Änderung ihrer refraktiven Eigenschaften ist die Lasik (Laser in-situ Keratomileusis). Bei der Lasik wird aus der Kornea ein oberflächliches Deckelscheibchen herausgeschnitten, das in der Fachwelt gemeinhin als Flap bezeichnet wird. Der Flap hängt an einem Teil seines Rands in einem Scharnierbereich (englisch: hinge) noch an dem daneben liegenden Hornhautgewebe, sodass er problemlos zur Seite geklappt und später wieder zurückgeklappt werden kann. Zur Erzeugung des Flaps kommen in der bisherigen Praxis insbesondere zwei Methoden zum Einsatz, zum einen eine mechanische mittels eines Mikrokeratoms und zum anderen eine lasertechnische, bei der mittels Femtosekunden-Laserstrahlung (d.h. gepulster Laserstrahlung mit einer Pulsdauer im fs-Bereich) ein flächiger Tiefenschnitt in die Kornea eingebracht wird, der ausgenommen des Scharnierbereichs zur Korneaoberfläche herausgeführt wird. Nach Wegklappen des erzeugten Flaps erfolgt ein Materialabtrag (Ablation) vom so freigelegten Stroma gemäß einem vorbestimmten Ablationsprofil. Das Ablationsprofil gibt an, an welcher Stelle der Kornea wie viel Gewebe abzutragen ist. Es wird so berechnet, dass nach der Ablation die Kornea eine für das behandelte Auge optimale Form hat und die zuvor vorhandenen optischen Abbildungsfehler des Auges weitestmöglich korrigiert sind. Für die Berechnung des Ablationsprofils stehen der Fachwelt seit längerem geeignete Methoden zur Verfügung. Für die Ablation kommt beispielsweise ein Excimer-Laser mit einer Strahlungswellenlänge im UV-Bereich zum Einsatz, etwa bei 193 nm.

Ist für das zu behandelnde Auge das Ablationsprofil bestimmt, wird anschließend berechnet, wie der gewünschte Abtrag mit der zur Verfügung stehenden Laserstrahlung (Behandlungsstrahlung) am besten erzielt werden kann. Die verwendete Laserstrahlung ist regelmäßig gepulste Strahlung. Es geht deshalb darum, eine Folge von Laserpulsen nach Raum und Zeit zu berechnen, die in Wechselwirkung mit der Kornea, insbesondere dem Stroma, die gewünschte Neuformung der Kornea bewirkt.

Strahlführungsmittel, um einen Laserstrahl so über das zu behandelnde Auge zu führen, dass sich die gewünschte Raum- und Zeitfolge der Laserpulse einstellt, sind als solche im Stand der Technik bekannt. Insbesondere können die Strahlführungsmittel eine der Ablenkung des Behandlungslaserstrahls in Querrichtung (x-y-Richtung) dienende Ablenkeinheit, bekannt auch als Scanner, sowie eine Fokussieroptik zur Fokussierung des Laserstrahls an einer gewünschten Höhenposition (z-Position) umfassen. Die Ablenkeinheit kann beispielsweise einen oder mehrere galvanometrisch gesteuerte Ablenkspiegel umfassen.

Die erwähnten Strahlführungsmittel werden mittels eines programmgesteuerten Rechners nach Maßgabe des Ablationsprofils gesteuert. Da die Erfindung keineswegs auf eine Anwendung bei der Lasik beschränkt ist, sondern bei zahlreichen anderen laserchirurgischen Eingriffen am Auge zum Einsatz kommen kann, sei im Folgenden allgemein von einem Bearbeitungsprofil gesprochen, nach dessen Maßgabe die Strahlführungsmittel gesteuert werden. Für inzisionale Eingriffe, bei denen Schnitte in die Hornhaut oder eine andere Komponente des Auges eingebracht werden, kann das Bearbeitungsprofil ein Schnittprofil darstellen, das angibt, an welcher Stelle wie tief ein Schnitt zu setzen ist.

Das menschliche Auge ist kein ruhendes Objekt, sondern führt ständig Bewegungen aus. Es gibt unterschiedliche Arten von Augenbewegungen, die zum Teil auf unterschiedlichen Zeitskalen und mit unterschiedlichen Amplituden ablaufen. Wichtig ist allein die Feststellung, dass das Auge niemals stillsteht. Dies gilt auch dann, wenn versucht wird, den Blick auf ein bestimmtes vorgegebenes Objekt zu fixieren; selbst dann treten unvermeidliche Fixationsbewegungen auf.

Zur Erfassung der erwähnten Augenbewegungen sind Systeme zur Augenbewegungsverfolgung bzw. Blickbewegungsregistrierung (Eye-Tracker) bekannt. Diese Systeme umfassen regelmäßig wenigstens eine Kamera, welche auf das Auge gerichtet ist und Sequenzen von Bildern der Pupille einschließlich der umgebenden Iris aufnimmt. Durch anschließende Auswertung der Bildsequenzen mittels geeigneter Bildanalysealgorithmen kann die aktuelle Lage der Pupille und der Bewegungsverlauf der Pupille festgestellt werden. Insbesondere wird hierbei das Pupillenzentrum herangezogen. Durch Ausrichtung des Bearbeitungsprofils an dem so kameratechnisch überwachten Pupillenzentrum oder einem hiervon abgeleiteten Punkt kann trotz der unvermeidlichen Augenbewegungen die gewünschte Raumfolge von Laserpulsen zuverlässig auf die richtigen Stellen des zu behandelnden Augenbereichs gelenkt werden.

Grundlage für die Ermittlung eines geeigneten Bearbeitungsprofils ist regelmäßig eine Vermessung des Auges in seinem Ist-Zustand. Für die refraktive Korneabehandlung beispielsweise ist regelmäßig eine Kenntnis zumindest der Topographie und Dicke der Kornea erforderlich. Es kann die Kenntnis anderer oder weiterer Parameter des Auges für die durchzuführende Behandlung erforderlich sein, etwa der Vorderkammertiefe, der Linsendicke, der Gesamttiefe des Auges und dergleichen. Derartige Parameter werden nicht nur vor Beginn der Behandlung gemessen, sondern zumindest teilweise auch während oder/und nach der Behandlung, beispielsweise um den Behandlungsverlauf zu protokollieren und gegebenenfalls steuernd in den Behandlungsverlauf einzugreifen und um das Behandlungsergebnis zu überprüfen.

Seit einiger Zeit stehen zur berührungslosen Vermessung von Augenparametern, wie insbesondere der Hornhautdicke, kohärenzoptische interferometrische Messeinrichtungen zur Verfügung, die beispielsweise nach dem Prinzip der optischen Kurzkohärenzreflektometrie (OLCR: Optical Low-Coherence Reflectometry) oder der optischen Kohärenztomographie (OCT: Optical Coherence Tomography) arbeiten. Diese Messeinrichtungen arbeiten mit kurzkohärenter, breitbandiger Strahlung und gestatten es, Strukturen des Auges (oder allgemein des zu vermessenden biologischen Gewebes) mit einer Auflösung im Bereich von 1 µm und feiner zu vermessen. Die optische Kohärenztomographie ist ein bildgebendes Verfahren, das die Erzeugung von Schnittbildern ermöglicht. Die optische Kurzkohärenzreflektometrie eignet sich dagegen besonders für punktuelle Messungen eines Dicken- oder Tiefenmaßes des Auges, wie etwa der Hornhautdicke (Pachymetrie).

Die Messung der Korneadicke (oder eines anderen Dicken- oder Tiefenmaßes des Auges) wird durch die oben erwähnten Augenbewegungen erschwert. Sind mehrfache Messungen der Korneadicke erwünscht, etwa während einer laufenden Operation, so sollte die Messung möglichst stets am selben Punkt der Kornea oder zumindest innerhalb eines bestimmten Bereichs der Kornea (Akzeptanzbereich) erfolgen, in dem verlässliche Messergebnisse erwartet werden können. Fixationsbewegungen des Auges können jedoch dazu führen, dass dieser Akzeptanzbereich aus dem "Blickfeld" des pachymetrischen Messgeräts verschwindet und die Messung deswegen unterbrochen werden muss. Dies hat für den Arzt zur Folge, dass keine Messdaten erfasst werden können. Er muss dann entweder den Patientenkopf nachführen oder die Messung nicht weiter durchführen.

Aufgabe der Erfindung ist es, Dicken- oder Tiefenmessungen, die im Rahmen einer laserchirurgischen Behandlung des Auges anfallen, sei es vor, während oder nach der Operation, zu vereinfachen und zuverlässiger zu machen.

US2005/0024586 offenbart eine Vorrichtung gemäß der Präambel von Anspruch 1.

Zur Lösung dieser Aufgabe ist erfindungsgemäß eine Vorrichtung zur ophthalmologischen, insbesondere refraktiven Laserchirurgie nach Anspruch 1 vorgesehen, umfassend
- eine erste Strahlungsquelle zur Bereitstellung eines Behandlungslaserstrahls,
- erste Strahlführungsmittel zum orts- und zeitgesteuerten Führen des Behandlungslaserstrahls über ein zu behandelndes Auge,
- eine Kamera zur Aufnahme eines Bilds des zu behandelnden Auges,
- eine die Bilddaten der Kamera zur Erkennung von Augenbewegungen auswertende Auswerte- und Steueranordnung und
- eine kohärenzoptische interferometrische Messeinrichtung zur Messung eines Dicken- oder Tiefenmaßes, insbesondere einer Hornhautdicke, des Auges, wobei die Messeinrichtung eine einen Messstrahl bereitstellende zweite Strahlungsquelle sowie zweite Strahlführungsmittel umfasst, um den Messstrahl auf das Auge zu richten.

Erfindungsgemäß umfassen dabei die zweiten Strahlführungsmittel mindestens ein zur Veränderung der Strahlposition des Messstrahls beweglich angeordnetes Strahlführungselement, wobei die Auswerte- und Steuereinheit dazu eingerichtet ist, das Strahlführungselement abhängig von erfassten Augenbewegungen derart zu steuern, dass die Position des Messstrahls den Augenbewegungen folgt.

Die Erfindung lehrt somit den Gedanken, die Daten eines die Augenbewegungen erfassenden Eye-Trackers zur Steuerung der Messeinrichtung zu verwenden, so dass der Messstrahl bei der Durchführung der Messung stets im Wesentlichen auf denselben Punkt der Korneaoberfläche oder zumindest im selben Bereich der Korneaoberfläche auftrifft. Die solchermaßen automatisierte Nachführung des Messstrahls in Abhängigkeit von den erkannten Augenbewegungen ermöglicht eine hohe Vielzahl von Messungen in zeitlich kurzer Abfolge, was eine präzise Dokumentation oder/und Steuerung des Behandlungsablaufs erlaubt. Die Mühsamkeit der manuellen Nachführung bei bisherigen Systemen führt dagegen dazu, dass immer wieder große Zeitabstände zwischen aufeinander folgenden Messungen auftreten. Außerdem gewährleistet die erfindungsgemäße Lösung eine hohe Messzuverlässigkeit, da die Kopplung der Messeinrichtung an den Eye-Tracker eine gleichbleibend präzise Ausrichtung des Messstrahls auf einen vorgegebenen Punkt oder einen vorgegebenen Bereich des Auges gestattet.

Die Forderung, das Strahlführungselement der Messeinrichtung so zu steuern, dass die Position des Messstrahls den Augenbewegungen folgt, beinhaltet nicht eine fortlaufende, kontinuierliche Nachführung des Messstrahls an aktuell erfasste Augenbewegungen. Wie bereits erwähnt, ist ein Akzeptanzbereich vorgegeben , innerhalb dessen sich der Messstrahl auf der Korneaoberfläche befinden kann, ohne dass dies signifikante Auswirkungen auf die Messgenauigkeit hätte. Sobald der Messstrahl jedoch den Akzeptanzbereich verlässt, erfolgt eine Nachführung des Messstrahls, so dass dessen Position wieder innerhalb des Akzeptanzbereichs liegt. Hierbei können zusätzliche Randbedingungen zu erfüllen sein, die unnötige Nachführbewegungen des Messstrahls verhindern sollen. Beispielsweise kann eine derartige Randbedingung sein, dass der Messstrahl den erwähnten Akzeptanzbereich für mindestens eine vorbestimmte Zeitspanne verlassen haben muss, bevor eine Nachführung des Messstrahls erfolgt. Kurzzeitige Ausreißer können so herausgefiltert werden. Zumindest sollte sichergestellt sein, dass zu den Zeitpunkten, zu denen die Messeinrichtung eine Messung durchführt, der Messstrahl im Wesentlichen auf den vorgegebenen Punkt oder den Akzeptanzbereich ausgerichtet ist. Da die Nachführung des Messstrahls vergleichsweise rasch möglich ist, ist es vorstellbar, erst kurz vor der beabsichtigten Messung die Position des Messstrahls abhängig von der dann aktuellen Augen- bzw. Pupillenposition zu justieren. Es ist selbstverständlich ebenso möglich, Nachführungen des Messstrahls auch dann vorzunehmen, wenn gerade keine Messung unmittelbar bevorsteht.

Bei einer bevorzugten Ausführungsform ist das beweglich angeordnete Strahlführungselement ein Umlenkspiegel, von dem der Messstrahl ohne weitere Umlenkung an einem Spiegel auf das Auge gelangt. Sofern der Umlenkspiegel im Weg eines weiteren auf das Auge gerichteten Lichtstrahls der laserchirurgischen Vorrichtung liegt, ist der Umlenkspiegel zweckmäßigerweise ein teildurchlässiger Spiegel. Beispielsweise kann ein solcher weiterer Lichtstrahl ein von einer Fixationslichtquelle ausgesendeter Fixierlichtstrahl sein.

Zur Positionsveränderung des Messstrahls auf dem Auge kann der Umlenkspiegel um mindestens eine Kippachse kippbar angeordnet sein. Alternativ oder zusätzlich kann er längs mindestens einer Linearrichtung geradlinig verstellbar angeordnet sein.

Es wurde bereits erwähnt, dass es nicht in jedem Fall erforderlich ist, die Position des Messstrahls fortlaufend an jede erfasste Augenbewegung anzupassen. Dementsprechend ist gemäß der Erfindung die Auswerte- und Steuereinheit dazu eingerichtet, nur dann den Messstrahl durch Steuerung des Strahlführungselements den Augenbewegungen nachzuführen, wenn die erfassten Augenbewegungen mindestens eine vorbestimmte Bedingung erfüllen. Eine solche vorbestimmte Bedingung für das Nachführen des Messstrahls ist , dass sich das Auge um mindestens ein vorbestimmtes Maß gegenüber einer Vergleichsposition bewegt hat. Die Vergleichsposition kann sich beispielsweise auf die Lage des Pupillenzentrums beziehen. Marktgängige Eye-Tracker und ihre Bildauswertungssoftware sind in der Lage, aus den erfassten Bilddaten die aktuelle Position des Pupillenzentrums zu berechnen. Beispielsweise kann als eine erste Vergleichsposition die Lage des Pupillenzentrums zu Beginn der Laserchirurgie verwendet und der Messstrahl relativ zu dieser ersten Vergleichsposition ausgerichtet werden. Solange sich das Pupillenzentrum anschließend innerhalb eines vorgegebenen Bereichs (definiert beispielsweise durch einen vorgegebenen Radius) um die erste Vergleichsposition befindet, erfolgt keine Nachführung des Messstrahls. Wenn sich das Pupillenzentrum dagegen um mehr als den vorgegebenen Radius von der ersten Vergleichsposition entfernt, kann eine Nachführung des Messstrahls erfolgen. Dies kann beispielsweise in der Weise geschehen, dass eine neue Vergleichsposition auf Basis der aktuellen Informationen über die Lage des Pupillenzentrums festgelegt wird und der Messstrahl nun an der neuen Vergleichsposition ausgerichtet wird. Die neue Vergleichsposition kann beispielsweise eine gemittelte Position des Pupillenzentrums nach Verlassen des vorherigen Akzeptanzbereichs sein. Abhängig von der neuen Vergleichsposition wird dann ein neuer Akzeptanzbereich festgelegt, wiederum beispielsweise in Form eines Umkreises um die neue Vergleichsposition mit einem vorbestimmten Radius. Es versteht sich, dass andere Prozeduren und Bedingungen für die Nachführung des Messstrahls abhängig von den Bewegungen des Auges jederzeit möglich sind.

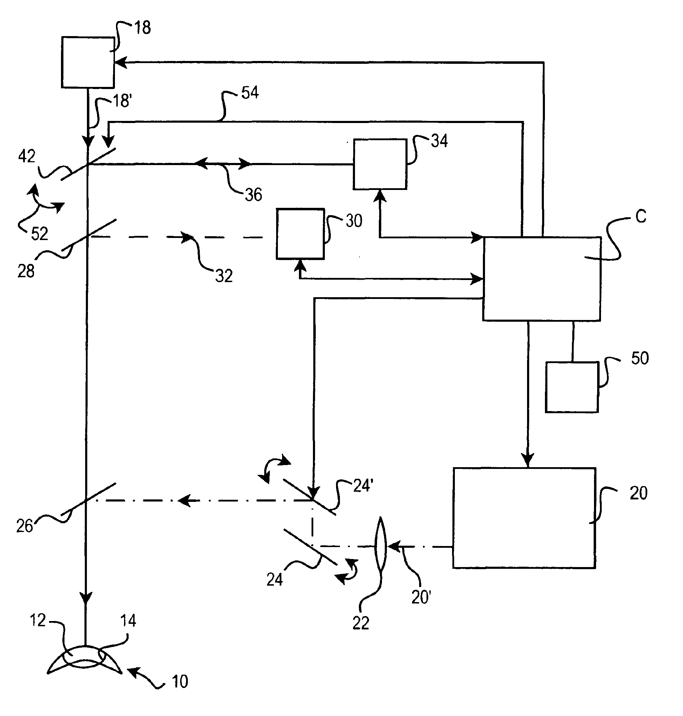

Die Erfindung wird nachfolgend anhand der beigefügten einzigen Zeichnung weiter erläutert. Diese zeigt in schematischer Blockdarstellung ein Ausführungsbeispiel einer Vorrichtung für die refraktive Laserchirurgie des Auges. In der Zeichnung ist ein mit Laserchirurgie, beispielsweise refraktiver Laserchirurgie, zu behandelndes Auge schematisch bei 10 angedeutet. Die Kornea des Auges 10 sowie der Pupillenrand sind bei 12 bzw. 14 gezeigt.

Die dargestellte laserchirurgische Vorrichtung weist in an sich bekannter Weise eine Fixationslichtquelle 18 auf, die einen (schwachen) Fixationslichtstrahl 18' emittiert und vom Patienten zur Fixierung des Auges anvisiert wird.

Ferner umfasst die laserchirurgische Vorrichtung einen Behandlungslaser 20, der Behandlungsstrahlung 20' emittiert, die über eine Linse 22 auf ein Paar Scanner-Spiegel 24, 24' gerichtet und über einen teildurchlässigen Umlenkspiegel 26 auf das Auge 10 gelenkt wird. Für eine Lasik-Behandlung kann der Laser 20 beispielsweise ein Excimer-Laser sein, dessen Strahlungswellenlänge im UV-Bereich liegt, etwa bei 193 nm. Es versteht sich, dass für andere Behandlungszwecke gewünschtenfalls auch andere Behandlungswellenlängen verwendet werden können, auch im InfrarotBereich. Die Scanner-Spiegel 24, 24' sind beispielsweise galvanometrisch steuerbar und werden zusammen mit dem Laser 20 von einem programmgesteuerten Rechner C gemäß einem zuvor berechneten Behandlungsprofil gesteuert. Der Rechner C stellt eine Auswerte- und Steuereinheit im Sinne der Erfindung dar.

Die laserchirurgische Vorrichtung besitzt darüber hinaus eine Einrichtung zur Verfolgung von Augenbewegungen (Eye-Tracker). Der Eye-Tracker umfasst eine Kamera 30, mit der über einen teildurchlässigen Umlenkspiegel 28 in Richtung eines Pfeils 32 Bilder des Auges, konkret der Pupille und der Iris, aufgenommen werden können. Die Bilddaten der Kamera 30 werden sodann in dem Rechner C mittels Bildanalysesoftware ausgewertet, um Bewegungen des Auges, die der Patient trotz der versuchten Blickfixierung auf den Fixationsstrahl 18' in der Regel nicht vermeiden kann, zu verfolgen. Die detektierten Augenbewegungen berücksichtigt der Rechner C bei der Steuerung der Scanner-Spiegel 24, 24', um so das Bearbeitungsprofil möglichst konstant gegenüber einem beispielsweise an der Korneaoberfläche liegenden vorgegebenen Referenzpunkt des Auges ausgerichtet zu halten.

In die laserchirurgische Vorrichtung ist zudem eine Messeinrichtung 34 für die optische Kurzkohärenzreflektometrie (OLCR: Optical Low-Coherence Reflectometry) integriert, die in an sich bekannter Weise eine Strahlungsquelle (z.B. SLED, ASE, Supercontinuum-Laser) enthält, deren Messstrahl über einen teildurchlässigen Umlenkspiegel 42 auf das Auge 10 gerichtet wird. Die Messeinrichtung 34 empfängt vom Auge 10 reflektierte Strahlung über den Umlenkspiegel 42 auf dem gleichen Weg, auf dem die Messstrahlung der Messeinrichtung 34 ausgesendet wird. Dies ist durch einen Doppelpfeil 36 veranschaulicht.

Die Messeinrichtung 34 misst mindestens einmal, vorzugsweise aber mehrere Male die Hornhautdicke und gewünschtenfalls einen oder mehrere andere Dicken- oder Tiefenmaße (z.B. die Vorderkammertiefe) des zu behandelnden Auges 10. Zweckmäßigerweise erfolgt eine Hornhautdickenmessung zumindest einmal vor Beginn der Laserchirurgie und ein weiteres Mal nach Abschluss der Laserchirurgie. Vorzugsweise finden auch während der Laserchirurgie fortlaufend Messungen statt, z.B. in vorgegebenen regelmäßigen Intervallen. Die Messeinrichtung 34 liefert ihre Messdaten an den Rechner C, der die Messergebnisse beispielsweise auf einer Anzeigeeinheit 50 zahlenmäßig oder/und grafisch darstellen kann. Bei Bedarf kann der Rechner C auch dazu eingerichtet sein, die Messergebnisse zu speichern und im Anschluss an die Operation den Ausdruck eines Messprotokolls zu bewirken, das die im Rahmen der aufeinanderfolgenden Messungen gewonnenen Ergebnisse enthält. Die Anzeige der Messergebnisse auf der Anzeigeeinheit 50 ist jedoch insofern vorteilhaft, als sie es dem Operateur erlaubt, unmittelbar den Behandlungsfortschritt zu kontrollieren. Bei Bedarf kann der Rechner C bzw. sein Steuerprogramm so eingerichtet sein, dass es für korrigierende Eingriffe des Operateurs empfänglich ist und den Behandlungsablauf entsprechend anpasst. Solche korrigierenden Eingriffe können beispielsweise über eine nicht näher dargestellte Eingabevorrichtung möglich sein, mit welcher der Rechner C gekoppelt ist.

Der Umlenkspiegel 42, über welchen der Messstrahl in den gemeinsamen Strahlenweg des Fixationslichts 18' und des Behandlungslaserstrahls 20' eingekoppelt wird, ist relativ zu den beiden anderen Umlenkspiegeln 26, 28 beweglich angeordnet, nämlich im vorliegenden Beispielfall schwenkbar, wie durch einen Doppelschwenkpfeil 52 angedeutet. Dabei ist der Umlenkspiegel 42 um mindestens eine zur x-y-Ebene der laserchirurgischen Vorrichtung im Wesentlichen parallele Kippachse schwenkbar. Die x-y-Ebene ist gemäß gängiger Vorstellung die zur Einfallrichtung des Behandlungslaserstrahls 20' (z-Richtung) normale Ebene. Durch Verkippen des Umlenkspiegels 42 um eine derart liegende Kippachse kann der auf das Auge 10 treffende Teil des Messstrahls verschwenkt und damit die Auftreffposition dieses Laserstrahls verändert werden. Zweckmäßigerweise ist der Umlenkspiegel 42 um zwei zueinander senkrechte, jeweils im Wesentlichen parallel zur x-y-Ebene liegende Kippachsen schwenkbar, so dass die Position des Messstrahls auf dem Auge 10 zweidimensional verändert werden kann. Zur Verstellung des Umlenkspiegels 42 können beispielsweise galvanometrische Stellmittel vorgesehen sein, wie sie für den Antrieb von Scanner-Spiegeln (etwa der Spiegel 24, 24') an sich in der Fachwelt bekannt sind. Andere Antriebstypen sind selbstverständlich nicht ausgeschlossen, etwa elektromotorische oder piezoelektrische. Die erwähnten Stellmittel des Umlenkspiegels 42 werden von dem Rechner C gesteuert, was in der Zeichnung durch eine Steuerverbindung 54 angedeutet ist.

Alternativ zu einer Verschwenkbarkeit des Umlenkspiegels 42 kann dieser auch in der x-y-Ebene verstellbar sein, ohne dabei seine Orientierung relativ zu der x-y-Ebene zu ändern. Auch auf diese Weise ist eine Verlagerung der Position des Messstrahls auf dem Auge bewirkbar.

Der Rechner C steuert den Umlenkspiegel 42 abhängig von der aus den Bilddaten der Kamera 30 ermittelten Pupillenposition, genauer der Lage des Pupillenzentrums. Die Position des Messstrahls kann so in Abhängigkeit von erfassten Augenbewegungen nachgeführt werden, was gewährleistet, dass die Dickenmessung stets in einem Bereich der Hornhaut stattfindet, der zuverlässige Aussagen über die Hornhautdicke gestattet. Um ein Zahlenbeispiel zu geben, müsste der Umlenkspiegel 42 unter der Annahme eines Abstands zwischen dem Auge 10 und dem Umlenkspiegel 42 von etwa 445 mm und einer Verlagerung des von dem Messstrahl anvisierten Korneapunkts um etwa 1,0 mm um etwa 0,065° verkippt werden, um die erforderliche Neigung des Messstrahls um 0,13° zu erreichen, die notwendig ist, damit der Messstrahl weiterhin im Wesentlichen auf denselben Korneapunkt trifft.

## Patentansprüche

1. Vorrichtung zur ophthalmologischen, insbesondere refraktiven Laserchirurgie, umfassend
- eine erste Strahlungsquelle (20) zur Bereitstellung eines Behandlungslaserstrahls (20'),
- erste Strahlführungsmittel (24, 24', 26) zum orts- und zeitgesteuerten Führen des Behandlungslaserstrahls über ein zu behandelndes Auge (10),
- eine Kamera (30) zur Aufnahme eines Bilds des zu behandelnden Auges,
- eine die Bilddaten der Kamera zur Erkennung von Augenbewegungen auswertende Auswerte- und Steueranordnung (C),
- eine kohärenzoptische interferometrische Messeinrichtung (34) zur Messung eines Dicken- oder Tiefenmaßes, insbesondere einer Hornhautdicke, des Auges, wobei die Messeinrichtung eine einen Messstrahl bereitstellende zweite Strahlungsquelle sowie zweite Strahlführungsmittel umfasst, um den Messstrahl auf das Auge zu richten,
wobei die zweiten Strahlführungsmittel mindestens ein zur Veränderung der Strahlposition des Messstrahls beweglich angeordnetes Strahlführungselement (42) umfassen und die Auswerte- und Steuereinheit (C) dazu eingerichtet ist, das Strahlführungselement abhängig von erfassten Augenbewegungen derart zu steuern, dass die Position des Messstrahls den Augenbewegungen folgt,
**dadurch gekennzeichnet, dass** die Auswerte- und Steuereinheit (C) dazu eingerichtet ist, nur dann den Messstrahl durch Steuerung des Strahlführungselements (42) den Augenbewegungen nachzuführen, wenn die erfassten Augenbewegungen mindestens eine vorbestimmte Bedingung erfüllen, wobei eine vorbestimmte Bedingung für das Nachführen des Messstrahls ist, dass sich das Auge um mindestens ein vorbestimmtes Maß gegenüber einer Vergleichsposition bewegt.

2. Vorrichtung nach Anspruch 1,
**dadurch gekennzeichnet, dass** sich die Vergleichsposition auf eine Lage des Pupillenzentrums des Auges bezieht.

3. Vorrichtung nach Anspruch 2,
**dadurch gekennzeichnet, dass** die Auswerte- und Steuereinheit (C) dazu eingerichtet ist, als eine erste Vergleichsposition die Lage des Pupillenzentrums zu Beginn einer Laserbehandlung zu verwenden.

4. Vorrichtung nach Anspruch 2 oder 3,
**dadurch gekennzeichnet, dass** die Auswerte- und Steuereinheit (C) dazu eingerichtet ist, eine neue Vergleichsposition auf Basis aktueller Informationen über die Lage des Pupillenzentrums festzulegen.

5. Vorrichtung nach Anspruch 4,
**dadurch gekennzeichnet, dass** die neue Vergleichsposition eine gemittelte Position des Pupillenzentrums nach Verlassen eines durch das vorbestimmte Maß bestimmten vorherigen Akzeptanzbereichs ist.

6. Vorrichtung nach einem der vorhergehenden Ansprüche,
**dadurch gekennzeichnet, dass** das vorbestimmte Maß durch einen Bereich mit einem vorgegebenen Radius um die Vergleichsposition bestimmt ist.

7. Vorrichtung nach einem der vorhergehenden Ansprüche,
**dadurch gekennzeichnet, dass** eine weitere Bedingung für das Nachführen des Messstrahls ist, dass das Auge einen durch das vorbestimmte Maß bestimmten Akzeptanzbereich um die Vergleichsposition für mindestens eine vorbestimmte Zeitspanne verlässt.

8. Vorrichtung nach einem der vorhergehenden Ansprüche,
**dadurch gekennzeichnet, dass** das beweglich angeordnete Strahlführungselement (42) ein insbesondere teildurchlässiger Umlenkspiegel ist, von dem der Messstrahl ohne weitere Umlenkung an einem Spiegel auf das Auge (10) gelangt.

9. Vorrichtung nach Anspruch 8,
**dadurch gekennzeichnet, dass** der Umlenkspiegel (42) um mindestens eine Kippachse kippbar angeordnet ist.

10. Vorrichtung nach Anspruch 8,
**dadurch gekennzeichnet, dass** der Umlenkspiegel linear verstellbar angeordnet ist.

## Claims

1. Apparatus for ophthalmological, in particular refractive laser surgery, comprising
- a first radiation source (20) for providing a treatment laser beam (20'),
- first beam-guidance means (24, 24', 26) for location-controlled and time-controlled guiding of the treatment laser beam over an eye (10) to be treated,
- a camera (30) for recording an image of the eye to be treated,
- an evaluating and control arrangement (C) evaluating the image data of the camera for the purpose of recognising eye movements,
- an optical-coherence interferometric measuring device (34) for measuring a thickness dimension or depth dimension, in particular a corneal thickness, of the eye, the measuring device including a second radiation source providing a measuring beam and also second beam-guidance means in order to direct the measuring beam onto the eye,
wherein the second beam-guidance means include at least one movably arranged beam-guidance element (42) for changing the beam position of the measuring beam and the evaluating and control unit (C) is configured to control the beam-guidance element in a manner depending on registered eye movements in such a manner that the position of the measuring beam follows the eye movements,
**characterised in that** the evaluating and control unit (C) is configured to track the measuring beam to the eye movements by control of the beam-guidance element (42) only when the registered eye movements satisfy at least one predetermined condition, wherein a predetermined condition for the tracking of the measuring beam is that the eye moves by at least a predetermined extent in relation to a reference position.

2. Apparatus according to Claim 1,
**characterised in that** the reference position refers to a position of the pupil centre of the eye.

3. Apparatus according to Claim 2,
**characterised in that** the evaluating and control unit (C) is configured to use the position of the pupil centre at the beginning of a laser treatment as a first reference position.

4. Apparatus according to Claim 2 or 3,
**characterised in that** the evaluating and control unit (C) is configured to determine a new reference position on the basis of current information on the position of the pupil centre.

5. Apparatus according to Claim 4,
**characterised in that** the new reference position is an averaged position of the pupil centre after leaving a previous acceptance region defined by the predetermined extent.

6. Apparatus according to any one of the preceding claims,
**characterised in that** the predetermined extent is defined by a region with a predefined radius around the reference position.

7. Apparatus according to any one of the preceding claims,
**characterised in that** a further condition for the tracking of the measuring beam is that the eye leaves an acceptance region defined by the predetermined extent around the reference position for at least a predetermined period of time.

8. Apparatus according to any one of the preceding claims,
**characterised in that** the movably arranged beam-guidance element (42) is, in particular, a partially transmitting deflecting mirror from which the measuring beam reaches the eye (10) without further deflection on a mirror.

9. Apparatus according to Claim 8,
**characterised in that** the deflecting mirror (42) is arranged to be capable of tilting about at least one tilt axis.

10. Apparatus according to Claim 8,
**characterised in that** the deflecting mirror is arranged to be linearly adjustable.

## Revendications

1. Dispositif pour la chirurgie ophtalmologique par laser, en particulier pour la chirurgie réfractive, comportant
- une première source de rayonnement (20) pour la mise à disposition d'un faisceau laser de traitement (20'),
- des premiers moyens de guidage de faisceau (24, 24', 26) pour guider dans l'espace et dans le temps un faisceau laser de traitement au-dessus d'un oeil à traiter (10),
- une caméra (30) pour l'enregistrement d'une image de l'oeil à traiter, une unité d'évaluation et de commande (C) évaluant les données d'image de la caméra pour reconnaître les mouvements de l'oeil,
- un dispositif de mesure (34) de cohérence optique interférométrique pour mesurer une dimension en épaisseur ou en profondeur de l'oeil, en particulier une épaisseur de la cornée, ledit dispositif de mesure comportant une deuxième source de rayonnement qui génère un faisceau de mesure et des seconds moyens de guidage de faisceau pour diriger le faisceau de mesure sur l'oeil,
lesdits seconds moyens de guidage de faisceau comportant au moins un élément de guidage de faisceau (42) disposé de manière mobile pour modifier la position du faisceau laser, et l'unité d'évaluation et de commande (C) étant aménagée pour commander de telle sorte l'élément de guidage de faisceau en fonction des mouvements de l'oeil captés que la position du faisceau de mesure suit le mouvement de l'oeil,
**caractérisé en ce que** l'unité d'évaluation et de commande (C) est aménagée pour que le faisceau de mesure ne suive les mouvements de l'oeil par commande de l'élément de guidage de faisceau (42) que si les mouvements de l'oeil captés remplissent au moins une condition prédéfinie, une telle condition pour le réajustement du faisceau de mesure étant donnée lorsque l'oeil se déplace d'au moins une valeur prédéfinie par rapport à une position de comparaison.

2. Dispositif selon la revendication 1,
**caractérisé en ce que** la position de comparaison se rapporte à une position du centre de la pupille de l'oeil.

3. Dispositif selon la revendication 2,
**caractérisé en ce que** l'unité d'évaluation et de commande (C) est aménagée de manière à utiliser la position du centre de la pupille de l'oeil comme première position de comparaison, au début du traitement laser.

4. Dispositif selon la revendication 2 ou 3,
**caractérisé en ce que** l'unité d'évaluation et de commande (C) est aménagée pour déterminer une nouvelle position de comparaison sur la base d'informations indiquant la position momentanée du centre de la pupille.

5. Dispositif selon la revendication 4,
**caractérisé en ce que** la nouvelle position de comparaison est une position moyenne du centre de la pupille après avoir quitté une zone d'acceptation précédemment déterminée par la valeur de mesure prédéfinie.

6. Dispositif selon l'une des revendications précédentes,
**caractérisé en ce que** la valeur de mesure prédéfinie est déterminée par une zone d'un certain rayon autour de la position de comparaison.

7. Dispositif selon l'une des revendications précédentes,
**caractérisé en ce qu'**une condition supplémentaire pour le réajustement du faisceau de mesure est que l'oeil quitte pour au moins un laps de temps prédéfini une zone d'acceptation déterminée par la valeur de mesure prédéfinie et située autour de la position de comparaison.

8. Dispositif selon l'une des revendications précédentes,
**caractérisé en ce que** l'élément de guidage de faisceau (42) disposé de manière mobile consiste en particulier en un miroir de renvoi partiellement transparent depuis lequel le faisceau de mesure arrive sur l'oeil (10) sans devoir passer par un miroir de renvoi supplémentaire.

9. Dispositif selon la revendication 8,
**caractérisé en ce que** le miroir de renvoi (42) est disposé inclinable autour d'au moins un axe d'inclinaison.

10. Dispositif selon la revendication 8,
**caractérisé en ce que** le miroir de renvoi est disposé de manière à être linéairement déplaçable.
